(19) Europäisches Patentamt
European Patent Office
Office européen des brevets

(11) EP 0 515 313 B1

(12) EUROPEAN PATENT SPECIFICATION

(45) Date of publication and mention of the grant of the patent:
**09.08.2000 Bulletin 2000/32**

(51) Int. Cl.[7]: **C07K 14/655**, A61K 51/02

(21) Application number: **92810381.1**

(22) Date of filing: **20.05.1992**

(54) **Somatostatin analogues containing chelating groups and their radiolabeled compositions**

Chelatbildende Gruppen enthaltende Somatostatin Analoge und deren radiomarkierten Zusammensetzungen

Analogues de somatostatine contenants des agents de chelation et leurs compositions radio-marquées

(84) Designated Contracting States:
**BE CH DE FR GB IT LI NL**

(30) Priority: **23.05.1991 GB 9111199**

(43) Date of publication of application:
**25.11.1992 Bulletin 1992/48**

(73) Proprietor: **Novartis AG**
**4058 Basel (CH)**

(72) Inventors:
- **Albert, Rainer**
  **CH-4051 Basle (CH)**
- **Krenning, Eric Paul**
  **NL-3062 JC Rotterdam (NL)**
- **Lamberts, Steven Willem**
  **NL-3055 GJ Rotterdam (NL)**
- **Pless, Janos**
  **CH-4054 Basle (CH)**

(56) References cited:
**WO-A-89/11475** **GB-A- 2 225 579**
**US-A- 4 678 667**

- **CANCER RESEARCH vol. 49 , 15 May 1989 pages 2639 - 2644 R.W.KOZAK ET. AL 'Nature of the Bifunctional Chelating Agent Used for Radioimmunotherapy with Yttrium-90 Monoclonal Antibodies: Critical Factors in Determining in Vivo Survival and Organ Toxicity'**
- **RADIOTHER.ONCOL. vol. 8, no. 2 , 1987 pages 129 - 135 E.LAVIE ET AL. 'Labeling of Sarcoma Associated Monoclonal Antibody with 111In, 67Ga and 125I'**
- **INT.J.RADIAT.ONCOL.BIOL.PHYS. vol. 16, no. 3 , 1989 pages 731 - 736 M.UDAYACHANDER ET AL. 'Tumor Imaging of Dalton's Lymphoma Using Antibody-Desferal-Ga-67 Complex'**
- **EUR.J.NUC.MED. vol. 16, no. 12 , 1990 pages 859 - 64 M.OSHIMA ET AL. 'Evaluation of Primary Lung Carcinoma Using Technetium 99m-Hexamethylpropylene Amine Oxime: Preliminary Clinical Experience'**
- **EUR.J.NUCL.MED. vol. 16, no. 1-4 , 1990 pages 239 - 98 A.T.IRVINE ET AL. 'An evaluation of 99m- HMPAO Uptake in Cerebral Gliomas a Comparision with X-ray CT'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

The present invention relates to polypeptides, process for their production, pharmaceutical preparations containing them and their use as a pharmaceutical, e.g. for treatment of somatostatin receptor positive tumors or as in vivo imaging agents.
GB-A-2,225,579 discloses somatostatin peptides bearing at least one chelating group which can be labelled for in vivo diagnostic and therapeutic applications. These compounds are capable of binding to somatostatin receptors, e.g. expressed or overexpressed by tumors or metastases.
The present invention provides new somatostatin peptide ligands bearing at least one chelating group which are suitable for e.g. γ- or β-radionuclide or positron labelling and have interesting binding affinity properties to target somatostatin receptors present on tumors. The chelating group may be attached either directly or indirectly through a spacer or bridging group to the somatostatin peptide.
According to the invention there is provided a compound of formula I

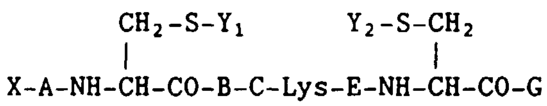

I

wherein

X is a radical of formula (a)

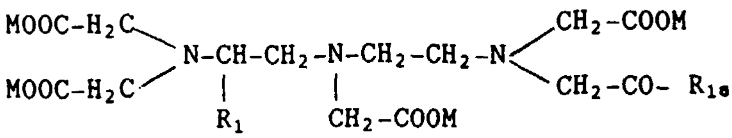

(a)

| | |
|---|---|
| M | is hydrogen or the equivalent of a pharmaceutically acceptable cation $R_1$ is hydrogen and $R_{1a}$ is a free bond |
| A | is (D)Phe, |
| B | is Phe ring substituted by hydroxy |
| C | is (D)Trp or Trp |
| E | is Thr, |
| G | is $-NH-CH(R_4)-X_1$ wherein $R_4$ is the radical attached to the α-carbon atom of Thr, and $X_1$ is $-CH_2OH$, and |
| $Y_1$ and $Y_2$ | represent together a direct bond |

the residues B and E having the L-configuration, the residues in the 2- and 7- positions having the L- or D-configuration.
The compounds of formula I may exist e.g. in free or salt form. Salts include acid addition salts with e.g. organic acids, polymeric acids or inorganic acids, for example hydrochlorides and acetates, and salt forms obtainable with the carboxylic groups present in the group X, e.g. those wherein M is an alkali metal cation such as sodium or potassium, or substituted or unsubstituted ammonium.
The present invention also includes a process for the production of the compounds of formula I. They may be produced by analogy to known methods.
The compounds of formula I may be produced for example as follows:

a) removing at least one protecting group which is present in a protected compound having the sequence indicated in formula I, or

b) linking together by an amide bond two peptide fragments each of them containing at least one amino acid or amino alcohol in protected or unprotected form and one of them containing the group X, wherein the amide bond is in such a way that the amino acid sequence indicated in formula I is obtained, and stage a) of the process is then optionally effected, or

c) linking together a chelating agent and a compound of formula II

```
      CH2-S-Y1        Y2-S-CH2
      |               |
H-A-NH-CH-CO-B-C-Lys-E-NH-CH-CO-G          II
```

in protected or unprotected form, and stage a) is then optionally effected or,

d) removing a functional group of an unprotected or a protected compound of formula I or converting it into another functional group so that another unprotected or protected compound of formula I is obtained and in the latter case stage a) of the process is effected, or
and recovering the compounds of formula I thus obtained in free form or in salt form.

The above reactions may be effected in analogy with known methods, e.g. as described in the following examples, in particular processes a) and c). Where desired, in these reactions, protecting groups which are suitable for use in peptides or for the desired chelating groups may be used for functional groups which do not participate in the reaction. The term protecting group may also include a polymer resin having functional groups.

The peptide fragment bearing the chelating group and used in stage b) may be prepared by reacting the peptide fragment comprising at least one amino acid or amino alcohol in protected or unprotected form with the chelating agent. The reaction may be performed in analogy with stage c).

The chelating agent used in process step c) may be known or prepared in analogy with known procedures. The compound used is such that it allows the introduction of the desired chelating group on the somatostatin peptide, e.g. it may also be used in form of an anhydride or in salt form.

The compounds of formula I may be purified in conventional manner, e.g. by chromatography.

The compounds of formula I in free form or in salt form are ligands. The chelating group X is capable to form a complex with a radionuclide and the corresponding labelled ligands are further capable of binding to somatostatin receptors, e.g. expressed or overexpressed by tumors or metastases.

Accordingly, the present invention also provides the compounds of formula I which are complexed with a positron, β- or γ-emitting radionuclide, in free form or in salt form, their preparation and their use for in vivo diagnostic and therapeutic treatment.

Suitable γ-emitting radionuclides include those which are useful in diagnostic techniques. The γ-emitting radionuclides advantageously have a half-life of from 1 hour to 40 days, preferably from 5 hours to 4 days, more preferably from 12 hours to 3 days. Examples are radionuclides derived from Gallium, Indium, Technetium, Yttrium, Ytterbium, Rhenium and Thallium e.g. $^{67}$Ga, $^{111}$In, $^{99m}$Tc, $^{90}$Y, $^{169}$Yb and $^{186}$Re. Preferably the γ-radionuclide is selected depending on the metabolism of the compound of formula I used. More preferably the compound of formula I is chelated with a γ-radionuclide having a longer half-life than the half-life of the non modified somatostatin peptide on the tumor. Suitable positron-emitting radionuclides are e.g. $^{68}$Ga. Suitable β-emitting radionuclides include those which are useful in therapeutic applications, e.g. as mentioned in GB-A-2,225,579 for example those derived from Y and Re. The β-radionuclide advantageously have a half-life of from 2.3 hrs to 14.3 d, preferably from 2.3 to 100 hrs. Preferably the β-emitting radionuclide is selected in order to have a longer half-life than the half-life of the non modified somatostatin peptide on the tumor.

Preferred are $^{111}$In, $^{90}$Y, $^{99m}$Tc and $^{68}$Ga.

The chelated compounds of formula I may be prepared e.g. as disclosed in GB-A-2,225,579 e.g. by reacting the compound of formula I with a corresponding radionuclide yielding compound, e.g. a metal salt, preferably a water-soluble salt.

Preferably the complexing of the compound of formula I is effected at a pH at which the compound of formula I is physiologically stable.

Radionuclides such as for example Technetium-99m may be used in oxidized form, e.g. Tc-99m pertechnetate, which may be complexed under reducing conditions.

The chelated compounds of formula I and their pharmaceutical acceptable salts exhibit pharmaceutical activity and are therefore useful either as an imaging agent, e.g. visualisation of an accumulation of somatostatin receptors, e.g. as in somatostatin receptor positive tumors and metastases, when complexed with a γ-emitting or positron-emitting radionuclide, or as a radiopharmaceutical for the treatment in vivo of tumours, particularly somatostatin receptor positive tumors and metastases when complexed with a β-radionuclide, as indicated by standard tests.

The affinity of the chelated compounds of formula I for somatostatin receptors expressed or overexpressed by tumors and metastases, has been determined in standard in vitro binding assays, e.g. as disclosed in GB-A-2,225,579.

The affinity of the chelated compounds of formula I for somatostatin receptors can also be shown by in vivo testing, according to standard test methods, e.g. as disclosed in GB-A-2,225,579.

After administration of a compound of formula I chelated with a γ-radionuclide, at a dosage of from 1 to 5 μg/kg of compound of formula I labeled with 0.1 to 2 mCi radionuclide the tumor site becomes detectable together with the organs where excretion essentially takes place.

Accordingly, in a series of specific or alternative embodiments, the present invention also provides:

1. A method for in vivo detection of tumors, particularly somatostatin receptor positive tumors or metastases in a subject which comprises a) administering a compound of formula I chelated with a γ- or positron-emitting radionuclide to said subject and b) recording the localisation of the tumors targeted by said chelated compound.

   The chelated compounds of formula I for use as an imaging agent may be administered parenterally, preferably intravenously, e.g. in the form of injectable solutions or suspensions, preferably in a single injection. The appropriate dosage will of course vary depending upon, for example, the compound of formula I and the radionuclide used. A suitable dose to be injected is in the range to enable imaging by photoscanning procedures known in the art. A chelated compound of formula I may advantageously be administered in a dose having a radioactivity of from 0.1 to 50 mCi, preferably 0.1 to 30 mCi, more preferably 0.1 to 20 mCi. An indicated dosage range may be of from 1 to 200 μg compound of formula I labeled e.g. with 0.1 to 20 mCi, preferably 3 to 15 mCi γ-emitting radionuclide, depending on the γ-emitting radionuclide used. For example with In, it is preferred to use a radioactivity in the lower range, whereas with Tc, it is preferred to use a radioactivity in the upper range.

   The enrichment in the tumorigenic sites with the chelated compounds of formula I may be followed by the corresponding imaging techniques, e.g. using nuclear medicine imaging instrumentation, for example a scanner, γ-camera , rotating γ-camera, each preferably computer assisted; PET-scanner (Positron emission tomography); MRI equipment or CAT scanning equipment.

2. A method for <u>in vivo</u> treatment of tumors, e.g. somatostatin receptor positive tumors and metastases in a subject in need of such a treatment which comprises administering to said subject a therapeutically effective amount of a compound of formula I chelated with a β-emitting radionuclide.

   Dosages employed in practising the therapeutic method of the present invention will of course vary depending e.g. on the particular condition to be treated, for exemple the volume of the tumor, the particular chelated compound employed, for exemple the half-life of the chelated compound of formula I in the tumor, and the therapy desired. In general, the dose is calculated on the basis of radioactivity distribution to each organ and on observed target uptake. For example the β-chelated compound of formula I may be administered at a daily dosage range having a radioactivity of from 0.1 to 3mCi/kg body weight, e.g. 1 to 3 mCi, preferably 1 to 1.5 mCi/kg body weight. An indicated dosage range is of from 1 to 200 μg compound of formula I labeled with e.g. 0.1 to 3 mCi/kg body weight, preferably 0.1 to 1.5 mCi/kg body weight β-emitting radionuclide.

   The β-emitting chelated compounds of formula I may be administered by any conventional route, in particular parenterally, e.g. in the form of injectable solutions or suspensions. They may also be administered advantageously by infusion, e.g. an infusion of 30 to 60 min. Depending on the site of the tumor, they may be administered as close as possible to the tumor site, e.g. by means of a catheter. The mode of administration selected may depend on the dissociation rate of the chelated compound used and the excretion rate.

The chelated compounds of formula I may be administered in free form or in pharmaceutically acceptable form. Such salts may be prepared in conventional manner and exhibit the same order of activity as the free compounds.

The chelated compounds of formula I for use in the method of the present invention may preferably be prepared shortly before the administration to a subject, i.e. the radiolabeling with the desired β-, γ- or positron-emitting radionuclide, may be performed shortly before the administration. The chelated compounds of formula I may be suitable for imaging or treating tumors such as pituitary, gastroenteropancreatic, central nervous system, breast, prostatic, ovarian or colonic tumors, small cell lung cancer, kidney cancer, paragangliomas, neuroblastomas, pheochromocytomas, lymphomas, medullary thyroid carcinomas, myelomas, Hodgkin' and non-Hodgkin's disease, bone tumors etc. and metastases thereof.

According to a further aspect of the invention, there is provided a pharmaceutical composition comprising a compound of formula I optionally chelated with a β-, γ- or positron-emitting radionuclide, in free or in pharmaceutically acceptable salt form, together with one or more pharmaceutically acceptable carriers or diluents therefor.

Such compositions may be manufactured in conventional manner.

A composition according to the invention may also be presented in separate package with instructions for mixing the compound of formula I with the radionuclide and for the administration of the resulting labeled compound. It may also be presented in twin-pack form, that is, as a single package containing separate unit dosages of the compound of formula I and the radionuclide with instructions for mixing them and for administration of the chelate. A diluent or carrier may be present in the unit dosage forms.

In the following examples, all temperatures are in ° C and $[\alpha]_D^{20}$ -values uncorrected. The following abbreviations are employed:

| | |
|---|---|
| Boc | tert.-butoxycarbonyl |
| TFA | trifluoroacetic acid |
| AcOH | acetic acid |
| DMF | dimethyl formamide |
| Fmoc | 9-fluorenylmethoxycarbonyl |

**EXAMPLE 1:**

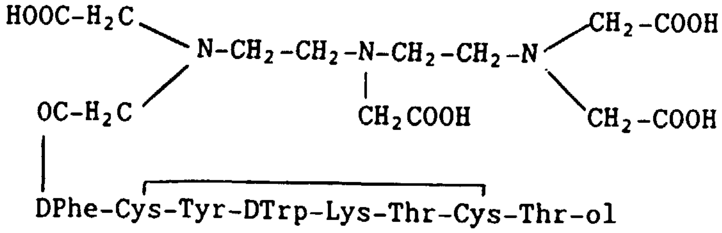

1.2 g of

H-DPhe-Cys-Tyr-DTrp-Lys(ε-Boc)-Thr-Cys-Thr-ol

in free base (1 mM), are dissolved in 5 l of dioxan/$H_2O$ 1/1 (v/v) and reacted with 5 g $NaHCO_3$. 520 mg DTPA dianhydride is slowly added with stirring. The reaction mixture is stirred for a further 30 min and dry-frozen. The residue is dissolved in 250 ml water and the pH is adjusted to pH 2.5 with concentrated HCl. The precipitated product is filtered out, washed and dried over phosphorus pentoxide. After cleavage of the Boc group by treatment with TFA, chromatography on a silica-gel column and desalting on a Duolite resin, the title compound is obtained.

$[\alpha]_D^{20}$ = -6.4° (c = 0.25 in 95% AcOH)

**EXAMPLE 2:**

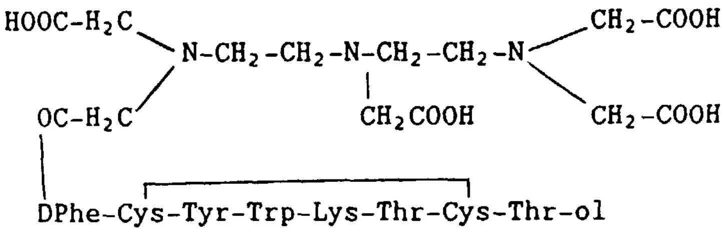

By repeating the procedure of Example 1 for coupling with DTPA but using

DPhe-Cys-Tyr-Trp-Lys(ε-Fmoc)-Thr-Cys-Thr-ol

and removing Fmoc by treatment for 10 minutes with piperidine/DMF (1/4 v/v) and removal of the solvent and the base, the title compound is obtained.

$[\alpha]_D^{20}$ = -3.4° (c = 0.25 in 95% AcOH)

**EXAMPLE 3:**

**$^{111}$In labeled**

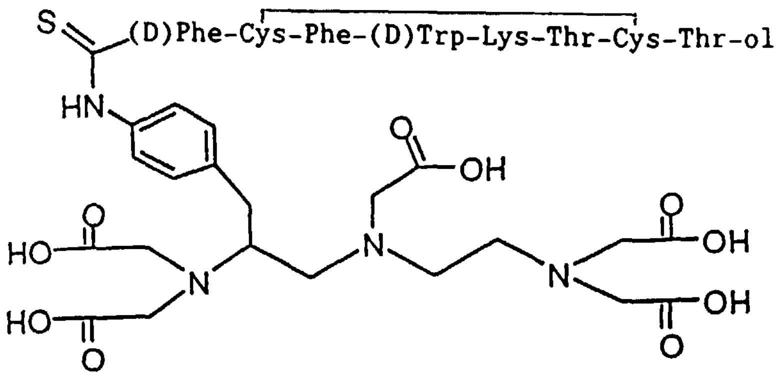

1 mg of the unlabelled compound is dissolved in 5 ml 0.01M acetic acid. The resulting solution is passed through a 0.22μ Millex-GV filter and dispensed in 0.1 ml portions and stored at -20°C. $^{111}InCl_3$ (Amersham, 1 mCi/100 μl) is prediluted in an equal volume of 0.5M sodium acetate and labeling is carried out by mixing the ligand with the $InCl_3$ solution and gentle homogenisation at room temperature.

HEPES buffer, pH 7.4, is then added to make a solution $10^{-6}$ M.

Compounds of Examples 1 and 2 are labeled with $^{111}$In in accordance with the procedure of Example 3.

**Reference EXAMPLE 4:**

**$^{90}$Y labeled**

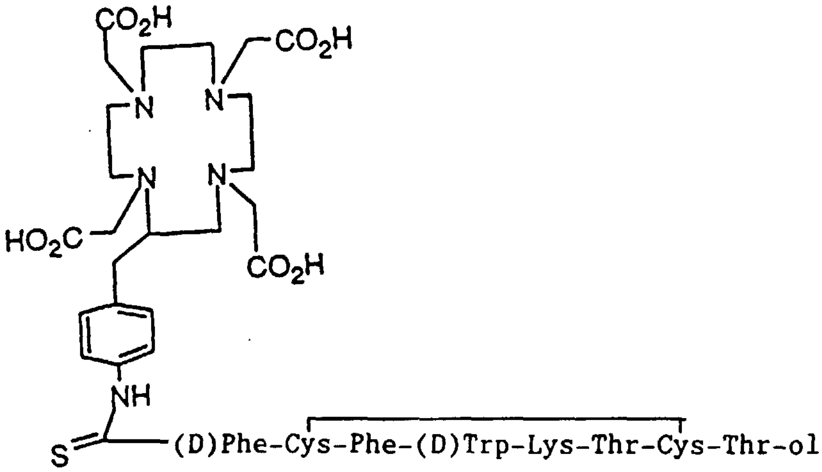

$^{90}$Y is obtained from a $^{90}$Sr-$^{90}$Y radionuclide generator. The construction of the generator, its elution and the conversion of the [$^{90}$Y]EDTA to the acetate complex are performed in accordance with the method disclosed by M.Chinol and D.J. Hnatowich in J. Nucl. Med. 28, 1465-1470 (1987). 1 mg of the unlabelled compound dissolved in 5ml 0.01M acetic acid is allowed to warm to room temperature and 1.0 mCi of $^{90}$Y in 50 μl sterile 0.5M acetate is added. The mixture is then left undisturbed for 30 min to 1 hr to maximize chelation.

Compounds of Examples 1 and 2 are labeled with $^{90}$Y in accordance with the procedure of Example 4.

**Claims**

1. A compound of formula I

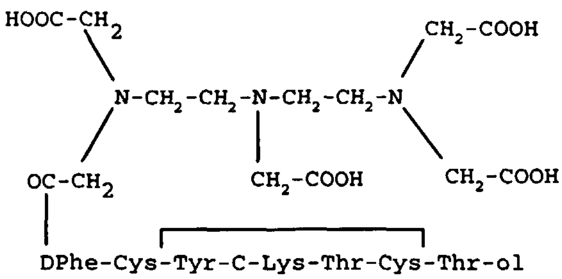

I

wherein

C is (D)Trp or Trp
in free form or in salt or complex form.

2. A compound of formula I according to claim 1 complexed with a β-, γ- or positron emitting radionuclide.

**3.** A compound of formula I according to claim 1 complexed with $^{111}In$ , in free form or in salt form.

**4.** A compound according to claim 2 or 3, in free form or in salt form, for use as a radiopharmaceutical.

**5.** A compound according to claim 3 in free form or in salt form, for use as an imaging agent to detect somatostatin receptor positive tumors and metastases.

**6.** A pharmaceutical composition comprising a compound according to any one of claims 1 to 3, in free form or in pharmaceutically acceptable salt form in association with a pharmaceutically carrier or diluent.

**7.** A package containing unit dosages of a compound of formula I in free form or in salt form according to claim 1 and of a γ- or positron emitting radionuclide with instructions for mixing them and for the use as imaging agent.

**Patentansprüche**

**1.** Verbindung der Formel I

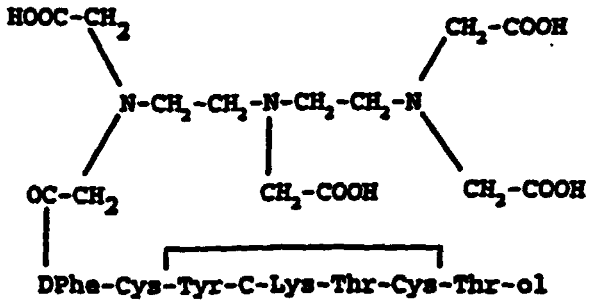

I

worin

C für (D)-Trp oder Trp steht,
in freier Form oder in Salz- oder Komplexform.

**2.** Verbindung der Formel I nach Anspruch 1, die mit einem ß-, γ- oder Positronen-emittierenden Radionuklid komplexiert ist.

**3.** Verbindung der Formel I nach Anspruch 1, die mit $^{111}In$ komplexiert ist in freier Form oder in Salzform.

**4.** Verbindung nach Anspruch 2 oder 3 in freier Form oder in Salzform zur Verwendung als radioaktives Pharmazeutikum.

**5.** Verbindung nach Anspruch 3 in freier Form oder in Salzform zur Verwendung als bildgebendes Mittel zur Detektion von Somatostatinrezeptor-positiven Tumoren und Metastasen.

**6.** Pharmazeutische Zusammensetzung, die eine Verbindung nach einem der Ansprüche 1 bis 3 in freier Form oder in pharmazeuzisch annehmbarer Salzform zusammen mit einem pharmazeutischen Träger oder Verdünmungsmittel enthält.

**7.** Packung, die Einheitsdosierungsformen einer Verbindung der Formel I in freier Form oder in Salzform nach Anspruch 1 und eines γ- oder Positronen-emittierenden Radionuklids mit Anleitungen zum Mischen der beiden und zur Verwendung als bildgebendes Mittel enthält.

**Revendications**

**1.** Composé de formule I

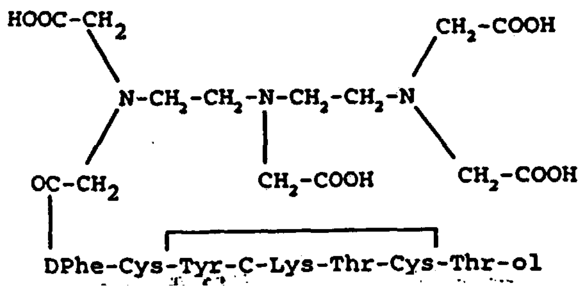

I

dans laquelle

C représente (D)Trp ou Trp
sous forme libre ou sous forme de sel ou de complexe.

**2.** Composé de formule I selon la revendication 1 complexé avec un radionucléide émetteur β, γ ou de positrons.

**3.** Composé de formule I selon la revendication 1 complexé avec $^{111}In$, sous forme libre ou sous forme de sel.

**4.** Composé selon la revendication 2 ou 3, sous forme libre ou sous forme de sel, pour utilisation comme substance radiopharmaceutique.

**5.** Composé selon la revendication 3 sous forme libre ou sous forme de sel, pour utilisation comme agent de formation d'images afin de détecter les tumeurs et métastases positives vis-à-vis des récepteurs de la somatostatine.

**6.** Composition pharmaceutique comprenant un composé selon l'une quelconque des revendications 1 à 3, sous forme libre ou sous forme de sel pharmaceutiquement acceptable en association avec un véhicule ou diluant pharmaceutique.

**7.** Conditionnement contenant des doses unitaires d'un composé de formule I sous forme libre ou sous forme de sel selon la revendication 1 et d'un radionucléide émetteur γ ou de positrons avec des instructions pour les mélanger et pour leur utilisation comme agent de formation d'images.